# EUROPEAN PATENT APPLICATION

(11) **EP 2 172 242 A1**
(43) Date of publication of application: **07.04.2010**
(21) Application number: 08165870.0
(22) Date of filing: 03.10.2008
(51) Int. Cl.: A61M 29/02, A61B 17/22, A61F 2/86

(54) **Intravascular Treatment Device**

(71) Applicant: National University of Ireland Galway, Galway (IE)
(72) Inventor: Blowick, Ray, Galway (IE); Murphy, Ph.D., Bruce, Galway (IE)
(74) Representative: Lane, Cathal Michael

(57) **Abstract**

A collapsible treatment device (81) for treating a target area of a vessel wall within a human or animal body, having a mesh portion (89), preferably annular in shape and with a contracted configuration allowing travel within the vessel to the target area and an expanded configuration allowing treatment of the target area. A treatment implement (84) is on the mesh portion and a protuberance either as part of the mesh or provided on a sheath which protects the vessel from contact with the implement during travel within the vessel.

## Description

### Field of the Invention

The present invention relates to medical devices. In particular, the invention relates to a catheter-based medical device for the treatment of internal body cavities including vessels such as arteries/veins or other hollow organs.

### Background to the Invention

There are many medical reasons to insert a medical device into the human body. To make a procedure during which a medical device is inserted minimally invasive it is desirable to use a device which does not require major surgical incision. Many devices are known which dilate or cut or score or inject at target sites within the body.

Often times to reduce trauma a medical device is inserted through an existing vessel in the body. For certain vessels no incision is required. For other vessels such as those forming the vasculature of the body, a minor incision is required to allow entry into an appropriate vessel. Once in an appropriate vessel the medical device may then be advanced along the vessel to a target site. The function of the medical device can then be used at the target site. Such functions include delivery of active substances, delivery of medical devices such as stents, dilation, scoring, abrasion, cutting, injection etc.

One target site which is often treated with such minimally invasive devices are lesions, including calcified lesions, and partial occlusions and full occlusions. Target sites may be found in any part of the vasculature including at bifurcation locations. Often coronary or peripheral arteries are treated for such lesions.

Many medical devices of the type described above are known and have been produced for particular end functions. Many are catheter-based devices.

In certain situations it may not be appropriate to implant a stent and a stand-alone dilation procedure may be preferred. Accordingly it has become desirable to provide devices which can obviate the requirement for a stent. Due to the benefit of treatment of any site being lost over time it is desirable to provide devices which are effective in preventing regression of an improved condition (such as improved luminal gain) at a treated target site. Additionally, in other situations a conventional angioplasty dilation catheter may not be capable of dilating a calcified (or difficult) target site and a specialist device that focuses the angioplasty force may be required for a successful procedure.

US Patent Publication No. 2004/0143287 describes a plaque-scoring catheter with a balloon on a distal end thereof. The device is said to comprise a non-axial scoring shell. This device is commercially available under the name AngioSculpt^{™} from Angioscore^{™} which is a ballon catheter-based device which includes nitinol wires each with angular corners which form scoring elements. The device is inflated to dilate vessels and to score lesions in the vessels. The device is said to increase luminal gain to a greater extent than by standard balloon dilation alone. The patent publication describes the scoring structure as being potentially a slotted cage. One embodiment describes expanding arings about a balloon. An embodiment is described where there are scoring implements on a trilobal balloon. There is still a danger of unintentional injury by the wires, cage or cutting implements or the blades on the balloon.

US Patent Publication No. 2007/0198047 describes a cutting balloon assembly with an inflatable balloon fixed to a distal end of a catheter. The device includes a covering which can expand and which has an array of cutting edges. The covering extends about and is raised above the outer surface of the balloon. The covering is described as being made from plastic or metal or a mesh of flexible woven polyurethane fibres. The covering expands with inflation of the balloon and contracts with deflation of the balloon. The covering is described as having a sharp outer surface which lacerates lesions etc. Given the sharp nature of the covering, even in the deflated state of the balloon, there is still a danger of injury along the travel path of the device within the body.

US Patent Publication No. 2005/0080478 describes a permanently implanted stent with cutting bars or blades. The ends of the blades are angled or bevelled to allow the blades to cut into blockages. There is still a danger of injury along the travel path within the body when inserting the stent.

US Patent Publication No. 2004/0215223 describes a cutting stent and balloon device wherein the balloon is provided with liquid crystal polymer fibrils to minimise longitudinal growth during inflation of the balloon. The balloon is employed to deliver the stent to a target area.

US Patent No. 7,279,002 describes a number of devices including an implantable stent with a cutting blade; a catheter with an inflatable balloon which has a cutting blade; and a sheath or sleeve which has a cutting blade. A sheath is described which can surround a stent. In certain embodiments at least a portion of the stent such as a portion of the blades are made from a shape-memory material. Blades are attached to the stent by adhesive or welding. A pre-programmed shape memory is described as useful for retraction of the blades from an expanded configuration. The blades are also discussed as being possibly contained within a valley or trough of the stent or the balloon. In certain embodiments a sheath is provided between the balloon and the stent to prevent premature opening out of the blades with inflation of the balloon. The sheath is retracted to allow stent opening. Cutting blades may also be provided on a sheath fitted about a balloon. The sheath may alternatively be left upon the implanted stent where it biodegrades.

US Patent Publication No. 2006/0085025 describes an angioplasty balloon with a non-deployable stent. A number of substantially axially-arranged wires each with a sinusoidal bend are disposed about a balloon. A connecting circumferential wire joins the wires about the balloon. In one embodiment u-shaped connectors are provided between adjacent wires. Expansion of the balloon causes expansion of the wires. The device, and in particular the wires, are adapted to crack occlusions in vessels.

US Patent Publication No. 2005/0021070 describes a device for stent manipulation and in particular expanding a cell of a stent for such as in part of a stent that is implanted in one vessel but extends across an opening within that vessel to a branched vessel. The device has a similar structure to that described in US Patent Publication No. 2004/0143287 (supra) with an additional overtube which is proximate the wires of the stent and which can be utilised to control the compliance of the balloon part of the device.

Notwithstanding the state of the art, it would still be desirable to provide alternative catheter-based medical devices for the treatment of internal body cavities including vessels such as arteries/veins or other hollow organs.

### Summary of the Invention

In one embodiment the present invention relates to a collapsible treatment device for treating a target area of a vessel wall of a vessel within a human or animal body, comprising:
(i) a mesh portion having a contracted configuration allowing travel within the vessel to the target area and an expanded configuration allowing treatment of the target area;
(ii) at least one treatment implement carried on the mesh portion; and further comprising
(iii) at least one (collapsible) protuberance which protects the vessel from contact with the implement during travel within the vessel.

Desirably said at least one treatment implement is secured to the mesh portion. Such an arrangement of the treatment implements on the mesh ensures that the treatment implements are firmly fixed in place and are much less likely to become disengaged, thus avoiding the problem of loss of the implement due to detachment during movement of the device. Complete or partial detachment of the implement is thus substantially reduced as the implement is directly held in place by the mesh. It will be appreciated that the treatment implement may be secured to the mesh portion, for example, by machine forming, welding, or fixing into an implement holder formed on said mesh portion. Alternatively, the implement may comprise a flared end or collar dimensioned to secure said implement in the mesh portion.

The at least one protuberance may be formed by the mesh portion. Desirably, the device of the present invention may comprise a plurality of protuberances formed by the mesh portion. Preferably, the mesh portion comprises a main body which is annular. Further desirably, the mesh portion may comprise at least one element which follows a tortuous path, the element resiliently deforming under expansion by decreasing tortuosity of its path.

In a further embodiment the device of the present invention may comprise a protective sheath for protecting the vessel from contact with the implement during travel within the vessel. Desirably, said at least one protuberance which protects the vessel from contact with the implement during travel within the vessel is provided on the sheath.

The at least one protuberance may deform to expose the implement in the expanded configuration of the device. Preferably, the protuberance may house the implement and the implement extends through the protuberance in the expanded configuration of the device.

The mesh portion of the device of the present invention may be embedded within the sheath. In an alternative embodiment the mesh portion may overlie the sheath. In yet a further embodiment the mesh portion may underlie the sheath. Desirably, the mesh portion overlies the sheath and the protuberance extends from the sheath past the mesh portion.

Preferably, the sheath is made of a resiliently deformable material such as an elastomeric material. The resilient expansion of the mesh provides additional bias towards the non-working configuration and thus the device of the invention returns to a compact arrangement for travel within a vessel when returned to the non-expanded configuration. When combined with a resilient expansion of the sheath the bias toward the non-working configuration is even greater.

The mesh portion of the device of the present invention may be machined from a tubular portion. Alternatively, the mesh portion may be formed by moulding.

In a further embodiment, the device of the present invention may comprise, for example, at least two annular mesh portions. A device comprising at least three annular mesh portions is also embraced by the present invention.
In such embodiments the mesh portions of the device of the present invention may be integrally formed. Desirably, each mesh portion forms a turn within a helix. Alternatively, said annular mesh portions may not be integrally formed but are joined by one or more separate elements. Said one or more separate elements may comprise a treatment implement or a carrier for a treatment implement. It will be appreciated that the treatment implement may be secured to said separate elements or said carriers, for example, by machine forming, welding, or fixing into an implement holder formed on said separate elements or said carriers.

The device of the present invention may further comprise an expandable portion such as a balloon and the mesh portion is fitted over the expandable portion. Desirably, the balloon may comprise a substantially cylindrical body with substantially conical end portions. In a further embodiment, the present invention provides for a device wherein the sheath forms an inflatable balloon.

In yet a further embodiment, the present invention provides for a collapsible treatment device for treating a target area of a vessel wall of a vessel within a human or animal body, the device comprising:
an expandable mesh portion which is expandable from a contracted configuration allowing travel within the vessel to the target area to an expanded configuration allowing treatment of the target area, the mesh portion being constructed of a shape memory material which has memory for the contracted configuration and
at least one treatment implement carried on the mesh portion.

As will be appreciated by a person skilled in the art said collapsible treatment device may have one or more of the additional features of the device of the present invention described herein (*vide supra*).

As used herein the term shape memory material is intended as a reference to a material capable of returning to its original contracted shape once the expanding force acting on the material has been removed.

The present invention also provides for an expandable mesh for use with a collapsible treatment device for treating a target area of a vessel wall of a vessel within a human or animal body, the mesh comprising:
an expandable mesh portion which is expandable from a contracted configuration allowing travel within the vessel to the target area, to an expanded configuration allowing treatment of the target area, and
at least one treatment implement carried on the mesh portion, the mesh portion further comprising at least one (collapsible) protuberance which protects the vessel from contact with the implement during travel within the vessel.

Also embraced by the present invention is an expandable mesh for use with a collapsible treatment device for treating a target area of a vessel wall of a vessel within a human or animal body, the mesh comprising:
(i) an expandable mesh portion which is expandable from a contracted configuration allowing travel within the vessel to the target area, to an expanded configuration allowing treatment of the target area;
(ii) at least one treatment implement carried on the mesh portion; and
(iii) a sheath provided on the mesh the sheath comprising at least one (collapsible) protuberance which protects the vessel from contact with the implement during travel within the vessel.

Desirably, the expandable mesh of the present invention is constructed of a shape memory material which has memory for the contracted configuration. Preferably, in an annular configuration, the expandable mesh of the present invention is capable of considerable diametric expansion relative to its contracted state.

In another aspect of the invention the implements are provided with a recession stop that prevents recession of the implement into the mesh and/or sheath and/or annular portion. For example the implement may settle into the device and not move to the same extent as the mesh. This means that the implement will not project past the mesh to the extent possible and thus reduce its effectiveness to be worked in a vessel. This retracted position is thus undesirable. By providing a recession stop such potential recession is avoided. The recession stop desirably is arranged to engage with the mesh. The mesh then provides sufficient support to prevent any reversion. In one arrangement the recession stop takes the form of one or more tabs. In another arrangement the recession stop takes the form of a pin.

The invention extends to a device, mesh and various assembles described herein with reference to and as illustrated in the accompanying drawings.

### Brief Description of the Drawings

**Figure 1** shows an end (sectional) view of an expandable mesh of the present invention.

**Figure 2** shows an end (sectional) view of an assembly comprising a mesh and sheath for inclusion in a device of the present invention.

**Figure 3** shows a similar view to Figure 2 and of a further embodiment of the invention which is similar to that of Figure 2.

**Figure 4** shows a similar view to Figures 2 and 3 of a further embodiment of the invention which is similar to that of those Figures.

**Figure 5** shows a flattened out top plan view of a mesh of the present invention.

**Figure 6** shows a flattened out top plan view of an alternative mesh arrangement to that of Figure 5.

**Figure 7** shows an enlarged view of the **part A** of the device of Figure 6.

**Figure 8** shows a perspective view of a mesh of the invention forming an annular shape and comprises a series of mesh portions.

**Figure 9** shows a perspective view of a device which is similar in construction to that of Figure 8.

**Figure 10** shows an enlarged view of the **part A** of the device of Figure 9.

**Figure 11** shows a perspective view of a further embodiment of a collapsible treatment device in the expanded configuration.

**Figure 12** shows a perspective view the mesh device of the invention applied to a balloon catheter device such as an angioplasty balloon device.

**Figure 13** shows an end sectional view of a mesh device of the invention with blades attached thereto.

**Figure 14** shows an end sectional view of a mesh device of the invention with needles attached thereto.

**Figure 15(a)****-(b)** shows two end-sectional views of a combined sheath/mesh device of the invention undergoing expansion (top to bottom) and contraction (bottom to top).

**Figure 16(a)****-(c)** shows respective side views of a blades of the invention;

**Figure 17** shows an enlarged view of part of the blade of Figure 16(b).

**Figure 18** shows an enlarged view of part of the blade of Figure 16(c).

**Figure 19** shows how the blade of Figure 16(b) and Figure 17 engages with a mesh.

**Figure 20** shows how the blade of Figure 16(c) and Figure 18 engages with a mesh.

### Detailed Description of the Drawings

One of the principal inventive concepts of the present invention is to provide a mesh which provides additional retractive force against the expandable portion (to move it towards the non-working configuration) in combination with one or more protective protuberances which protect one or more implements (such as blades or needles) during insertion toward the target site and which flatten to expose said implement(s) when the device is expanded to a working configuration. The protuberances are desirably on the sheath or on the mesh.

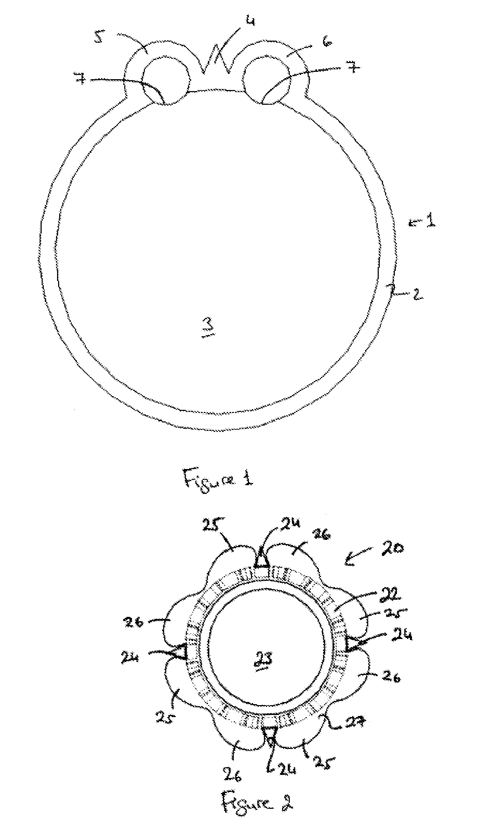

One embodiment of the present invention is shown in **Figure 1**. Figure 1 shows an end (sectional) view of an expandable mesh of the present invention. The mesh 1 comprises a mesh portion in the form of an annular body 2 which defines a cavity 3. An expandable portion such as a balloon (for example of a balloon catheter such as an angioplastly balloon) can be inserted in cavity 3. It is desirable that the mesh 1 is made from a shape memory material. In the embodiment the mesh is provided with a treatment implement in the form of a blade 4. The mesh 1 further comprises two protuberances 5,6 each on opposing sides of the blade. As shown in the embodiment it is desirable that the protuberances are integrally formed with the annular body 2. In particular as is shown it is desirable that the protuberances are also meshed. The two protuberances 5, 6 protect the blade 4 from contact with a vessel wall during insertion of the device. As will be described in more detail below for other embodiments, when the mesh is overfitted on a device and the cavity 3 is occupied by an expandable portion such as a balloon, expansion of the balloon causes the annular body 2 to expand and flatten, the protuberances in the form of ears or lobes 5, 6 will tend to flatten out thus exposing the blade 4 for treatment of a target site. The reverse process occurs with deflation when the protuberances 5, 6 return to their original shape to shield the treatment implement. The entire mesh 2, including protuberances 5, 6 is thus resiliently deformable with a bias toward the contracted configuration. If desired the device can further comprise one or more delivery reservoirs 7 formed within the protuberances. The delivery reservoirs 7 are for delivery of a medicament such as a therapeutic solution. The device can be configured so that the collapsing action of a protuberance expels the therapeutic solution. The device of Figure 1 could be adapted to have additional protuberances shielding one or more additional implements. These could for example be circumferentially arranged about the mesh 2. For example three sets approximately 120° apart may be utilised. It will be appreciated that the implement may be integrally formed with the mesh. However such an arrangement while desirable may be expensive. An alternative is to catch at least part of the implement, such as a base thereof, beneath the mesh. This may be achieved by partial insertion through the mesh. For example it may extend partially through the mesh from underneath. The problem of loss of the implement due to detachment during movement of the device is thus substantially reduced as the implement is directly held in place by the mesh.

A further embodiment of the present invention is shown in **Figure 2.** Figure 2 shows an end (sectional) view of an assembly for forming part of a device of the present invention. In this embodiment the assembly 20 comprises a mesh (or mesh portion) in the form of an annular body 22 which defines a cavity 23. An expandable portion such as a balloon (for example of a balloon catheter such as an angioplasty balloon) can be inserted in cavity 23. It is desirable that the mesh 22 is made from a shape memory material. The mesh 22 is embedded within a sheath 27. The sheath 27 is made of a resiliently deformable material such as an elastomeric material and is generally annular in shape. A plurality of implements are circumferentially spaced about the device. In the embodiment there are four implements approximately 90° apart, in the form of blades 24. The blades 24 are carried on the mesh 22 and are held in place by the mesh. This may be achieved in any suitable arrangement as set out above. For example the blade or a carrier therefor extends through the sheath 27 so that the mesh and the implement are connected by one or more connecting portions extending through the sheath 27.

In the embodiment the sheath 27 further comprises four pairs of protuberances in the form or ears or lobes 25,26 each protuberance on opposing sides of a respective blade 24. As shown in the embodiment it is desirable that the protuberances 25,26 are integrally formed with the annular body 22. In the embodiment the protuberances are non-hollow lobes. The protuberances collapse on expansion of the device to reveal the blades 24 as described for other embodiments.

The arrangement of the blades on the mesh ensure that the blades are firmly fixed in place and are much less likely to become disengaged. Furthermore the resilient expansion of the mesh provides additional bias towards the non-working configuration and thus the device of the invention returns to a compact arrangement for travel within a vessel when returned to the non-expanded configuration. When combined with a resilient expansion of the sheath the bias toward the non-working configuration is even greater.

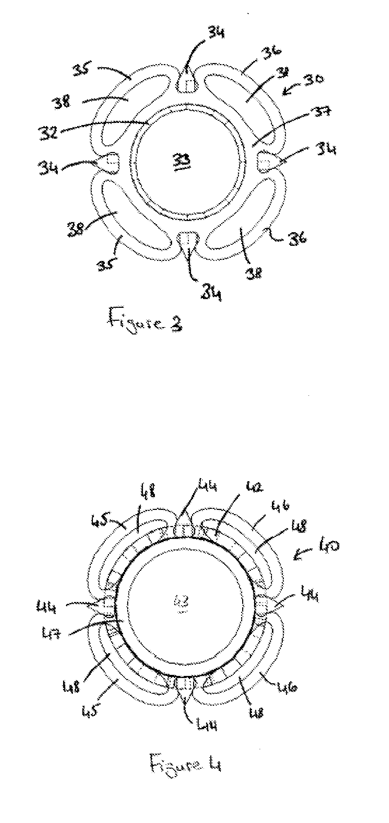

**Figure 3** shows a yet further embodiment of the invention which is similar to that of Figure 2. Figure 3 shows an end (sectional) view of an assembly for forming part of a device of the present invention. In this embodiment the assembly 30 comprises a mesh (or mesh portion) in the form of an annular body 32 which defines a cavity 33. An expandable portion such as a balloon (for example of a balloon catheter such as an angioplasty balloon) can be inserted in cavity 33. It is desirable that the mesh 32 is made from a shape memory material. The mesh 32 is positioned beneath a sheath 37. As with other embodiments the sheath 37 is made of a resiliently deformable material such as an elastomeric material. A plurality of implements are circumferentially spaced about the device. In this embodiment there are four implements approximately 90° apart, in the form of blades 34. The blades 34 are carried on the mesh 32 as described above for other embodiments. For example each blade or a carrier therefor extends through the sheath 37 so that each implement and the mesh are connected by one or more connecting portions extending through the sheath 37.

As above the sheath 37 further comprises protuberances in the form or ears or lobes 35,36 each protuberance on opposing sides of a respective blade 24. In fact in the embodiment there are two pairs of protuberances 35,36 and each extends between two blades to provide shielding for two blades at the same time. As shown in the embodiment it is desirable that the protuberances 25,26 are integrally formed with the sheath 37. In the embodiment the protuberances are (closed) hollow lobes providing an enclosed space 38 allowing a given enclosed space or protuberance to provide a conduit or reservoir. The protuberances collapse on expansion of the device to reveal the blades 34 as described for other embodiments. The collapsing action may also be used to expel therapeutic fluids as described above.

Again the arrangement of the blades on the mesh ensure that the blades are firmly fixed in place and are much less likely to become disengaged. Furthermore the resilient expansion of the mesh provides additional bias towards the non-working configuration and thus the device of the invention returns to a compact arrangement for travel within a vessel when returned to the non-expanded configuration. When combined with a resilient expansion of the sheath the bias toward the non-working configuration is even greater.

**Figure 4** shows an embodiment similar to those described above. An assembly 40 comprises a mesh (or mesh portion) in the form of an annular body 42 which defines a cavity 43 for an expandable portion such as a balloon. cavity 43. The mesh 42 is positioned above (and around) a sheath 47. As with other embodiments the sheath 47 is made of a resiliently deformable material such as an elastomeric material. A plurality of implements are circumferentially spaced about the device. In the embodiment there are four implements approximately 90° apart, in the form of blades 44. The blades 44 are carried on the mesh 42 as described above for other embodiments. The mesh sits close about the sheath 47.

As above the sheath 47 further comprises protuberances in the form of ears or lobes 45,46 each protuberance on opposing sides of a respective blade 44. In fact in the embodiment there are two pairs of protuberances 45,46 and each extends between two blades to provide shielding for two blades at the same time. As shown in the embodiment it is desirable that the protuberances 45,46 are integrally formed with the sheath 47. In the embodiment the protuberances are (closed) hollow lobes providing an enclosed space 48 allowing a given enclosed space or protuberance to provide a conduit or reservoir. The protuberances collapse on expansion of the device to reveal the blades 44 as described for other embodiments. The collapsing action may also be used to expel therapeutic fluids as described above.

In the embodiment the mesh 42 is outside the annular body of the sheath but does not extend beyond the protuberances. In this way the implements 44 are still shielded even though the mesh is external to the sheath. If desired the mesh can extend through the protuberances where they joint the sheath, but in many embodiments the mesh will not extend through the protuberances but extend about same.

Again the arrangement of the blades on the mesh ensure that the blades are firmly fixed in place and the resilient expansion of the mesh provides additional bias towards the non-working configuration.

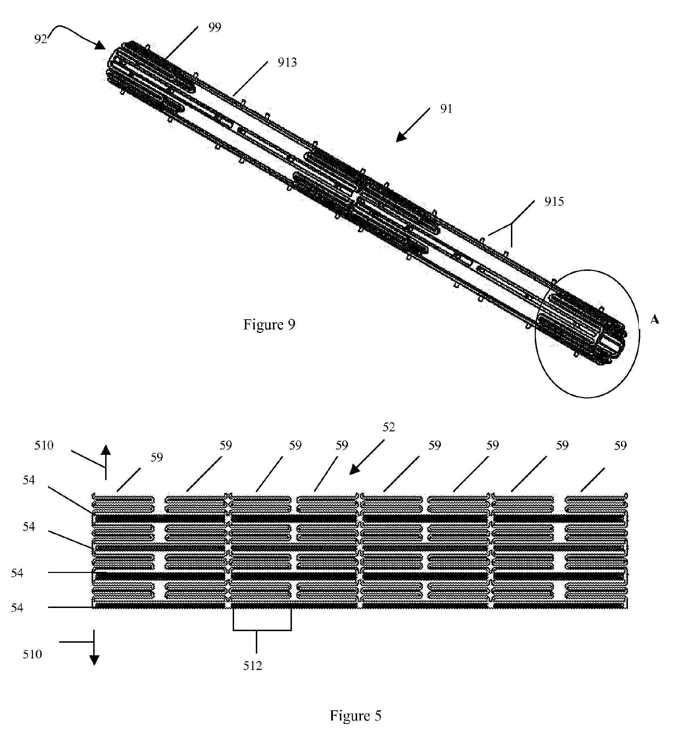

**Figure 5** shows one example of a mesh of the invention. In particular Figure 5 shows a flattened out top plan view of a mesh 52 of the present invention. The mesh 52 comprises a number of elements 59 each of which follows a tortuous path. In the embodiment the tortuous path is a path with a series of s-bends. The mesh is made from a resiliently deformable material and in particular a shape-memory material. Each element resiliently deforms under expansion (indicated by arrows 510) by decreasing tortuosity of its path. The distance between ends of the element increases as the element is extended.

A series of elements 59 are provided (in a side-by-side configuration) so as that the mesh 52 comprises a series of mesh portions 512 and is substantially continuous in a circumferential direction and an axial direction. Such an arrangement is suitable for applying to an expandable portion of a balloon catheter. It will be appreciated that the mesh 52 can be arranged in an annular manner for fitting over such an expandable portion. Implements, in the form of blades 54, are provided on the mesh. In the embodiment they, together with the elements 59, have been cut from a tubular piece. In other arrangements the implements can be welded to the mesh. Alternatively instead of implements being provided on the mesh directly implement holders can be provided. Such a mesh is suitable for use in any embodiment of the present invention. When utilised in the embodiment of Figure 1 the mesh will be formed to provide both an annular shape and one or more protuberances thereon for shielding the implement.

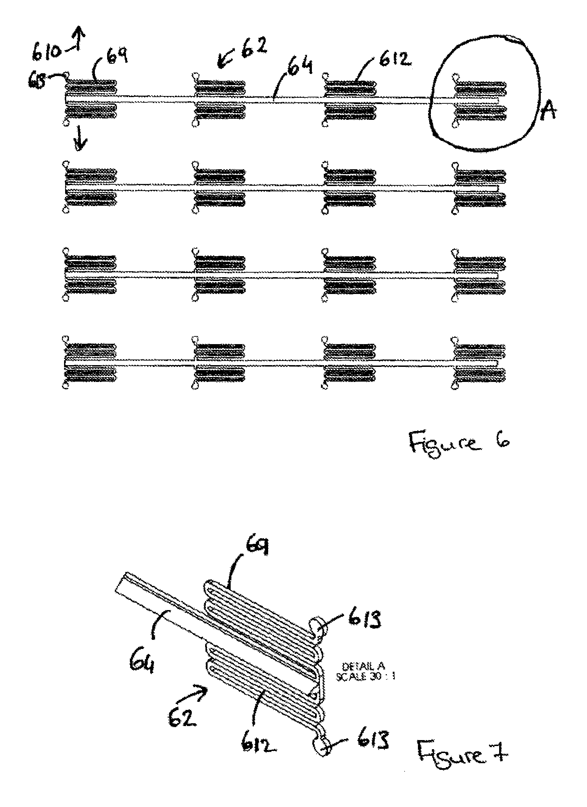

**Figure 6** shows an alternative mesh arrangement to that of Figure 5. In Figure 6 the mesh 62 comprises a series of discreet, spaced apart mesh portions 612. In particular the mesh portions are not integrally formed but are joined by one or more separate elements. Each portion 612 is formed by a tortuous element 69. In the embodiment an implement in the form of the blade 64 forms a support which joins the mesh portions together. The arrangement shown includes four discrete parts of the mesh. While such an arrangement may be used in any embodiment of the invention it is desirable to utilise such a mesh arrangement with an embodiment such as that described in Figure 2 with the mesh embedded in a sheath. To help with anchoring the mesh anchor points 613 are desirably provided on the free ends of the elements 69. As discussed in relation to Figure 5 the mesh portions can expand (circumferentially about the device) as indicated by arrows 610.

**Figure 7** is an enlarged view of the **portion A** of the device shown in Figure 6. In Figure 7 the mesh portion 612 is more clearly seen as is the tortuous path of the element 69.

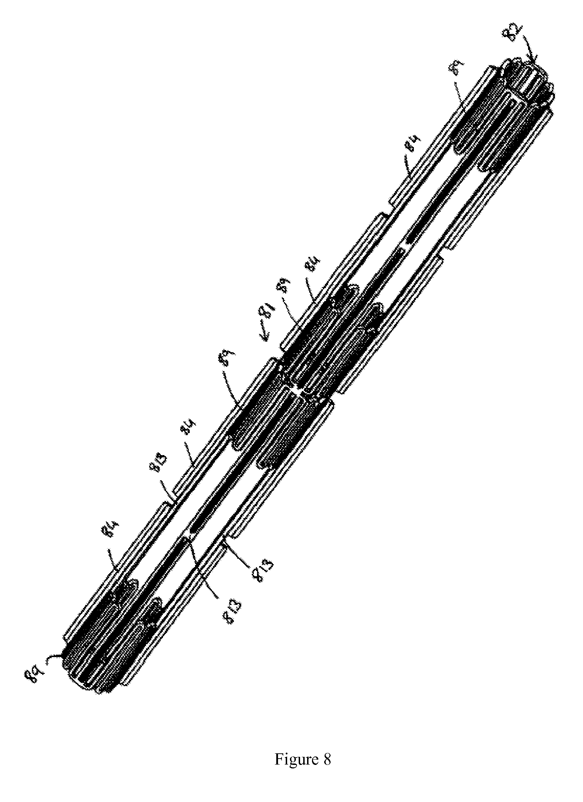

**Figure 8** shows a perspective view of a device 81 which is similar in construction to that of Figures 5-7. Figure 8 shows a mesh comprising an annular portion 82, which comprises a series of mesh portions 89 joined by a plurality of structural members 813 (in the embodiment there are four of them which are spaced 90° apart). The structural members 813 join the mesh portions together to form the annular arrangement shown. As is a desirable arrangement the structural members 813 carry the implements 84.

The arrangement of Figure 8 can be arranged so that the annular body is (i) buried in a sheath such as shown in Figure 2; (ii) beneath the sheath such as shown in Figure 3 or above the sheath such as shown in Figure 4. While the mesh layout differs from that of Figures 5 and 6 it will be appreciated that those mesh patterns are also suitable for forming an annular mesh arrangement. As with the other embodiments of the invention it is desirable that in a contracted configuration the implements are shielded by protuberances.

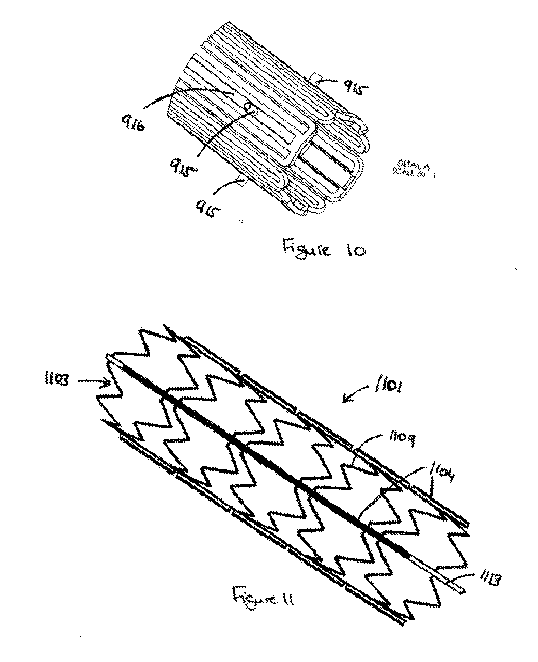

**Figure 9** shows a perspective view of a device 91 which is similar in construction to that of Figures 5-7 and in particular Figure 8. Figure 9 shows a mesh comprising an annular portion 92, which comprises a series of mesh portions 99 joined by a plurality of structural members 913 (in the embodiment 4 of them spaced 90° apart). The structural members 913 join the mesh portions together to form the annular arrangement shown. As is a desirable arrangement the structural members 913 carry the implements. In the embodiment the implements take the form of needles 915, in particular microneedles suitable for injecting fluids into the wall of a vessel in which the device is placed. An enlarged view of **portion A** of the device is shown in **Figure 10**.

The arrangement of Figure 9 can be arranged so that it is buried in a sheath, beneath the sheath or above the sheath. As with the other embodiments of the invention it is desirable that in a contracted configuration the implements are shielded by protuberances on the sheath.

As best seen from Figure 10 it is desirable that the device further comprises conduits which are in fluid communication with the needles 915 for example to supply a therapeutic fluid. In one simple construction seen in Figures 9 and 10 the conduits 916 are formed on or as part of the structural members 913.

**Figure 11** shows a further embodiment of a collapsible treatment device 1101 for treating a target area of a vessel wall of a vessel within a human or animal body. The device is similar in construction to previous embodiments but is shown in the expanded (working) configuration. In the expanded configuration each (s-shaped) element 1109 has opened out under expansive pressure from within a cavity 1103. This increases the distance between the respective loops in the tortuous path formed by member 1109 and results in a more zig-zag pattern. In the expanded configuration the bias of the members 1109 is toward the contracted configuration with tight looping of the member.

Again structural members 1113 are provided to join the mesh portions together and they also carry implements, in this case blades 1104. The implements are thus carried on a structural element of the mesh. The material forming the elements 1109 is desirably a material which has memory for the contracted configuration.

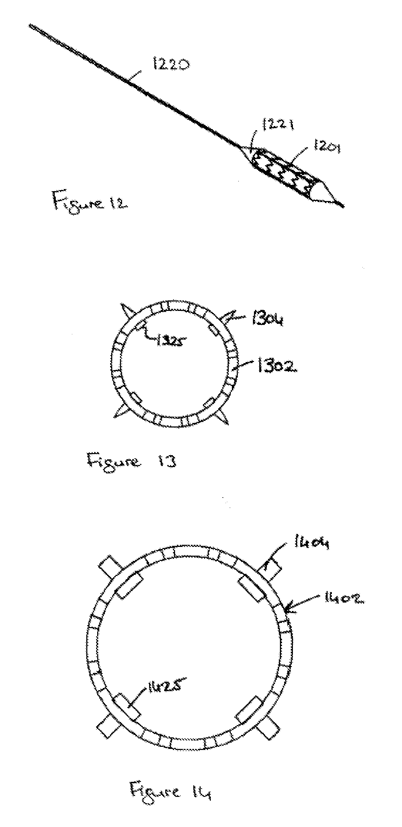

**Figure 12** shows the mesh device of the invention applied to a balloon catheter device such as an angioplasty balloon device. The device includes a catheter 1220, and expandable portion on the device in the form of a balloon 1221 and a collapsible treatment device 1201 which has a form similar to that of Figure 11. As with Figure 11 the device in Figure 12 is shown in an expanded configuration. Expansion of the balloon brings the device into its working configuration. Desirably the balloon catheter device will further comprise a sheath having protuberances which protect, in the non-working configuration, the implements, and which retract (flatten) to expose the implements for use in the working configuration thereof.

As with all embodiments of the invention, and in contrast to devices which are adapted to deploy, for example to deliver and deploy a stent in a constricted part of a vessel to open it up, the devices of the present invention and in particular the mesh or mesh portion forms a permanent part of the device (and is non-releasable). The device can travel to a target site, be expanded to a working configuration, used to treat the target site, return to a non-working or travel configuration under bias from the sheath and mesh, and be re-expanded to treat a further target site.

**Figure 13** shows one example of how blades 1304 can be fitted to a mesh 1302. The mesh is arranged in an annular shape. A base portion of each blade 1304 has a flared head or collar 1325 which is dimensioned to hold the blade 1304 to the mesh.

**Figure 14** shows a similar arrangement with one example of how needles 1404 can be fitted to a mesh 1402. The mesh is arranged in an annular shape. A base portion of each needle 1404 has a flared head or collar 1425 which is dimensioned to hold the needle 1404 to the mesh 1402. As described above the needles can be supplied with fluid through a conduit.

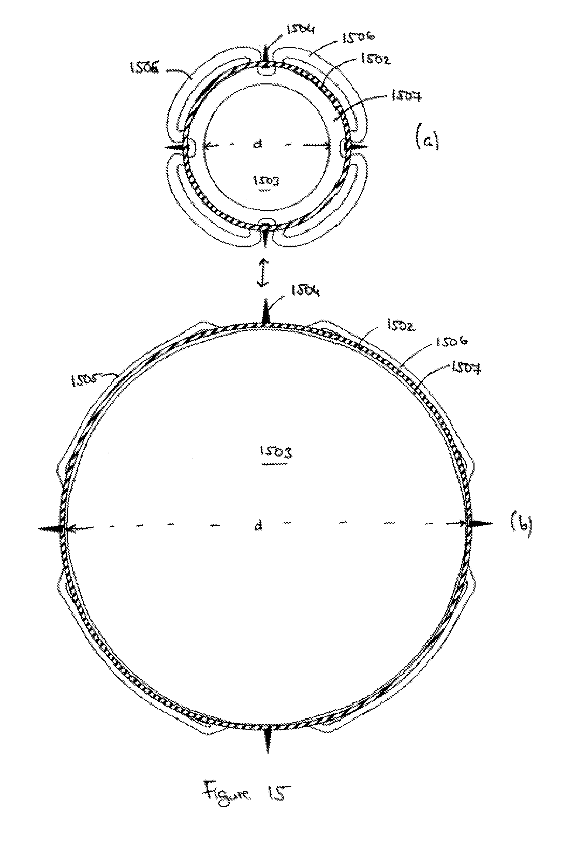

**Figure 15** shows expansion of a mesh and sheath (top to bottom sequence (a) to (b)) and the sequence for contraction (bottom to top sequence (b) to (a)). As indicated by the double-headed arrow expansion followed by contraction can be repeated as many times as is desirable.

In Figure 15 it can be seen that both the mesh 1502 and the sheath 1507 expand under an expansive force. This has a number of effects, firstly the diameter **d** of the cavity 1503 increases, the wall thickness of the sheath 1507 decreases, and the protuberances 1505,1506 flatten. These effects mean that the implements in the form of blades 1504 move closer to the vessel wall and undergo greater exposure [from their shielded position in (a) until they are fully exposed in (b)].

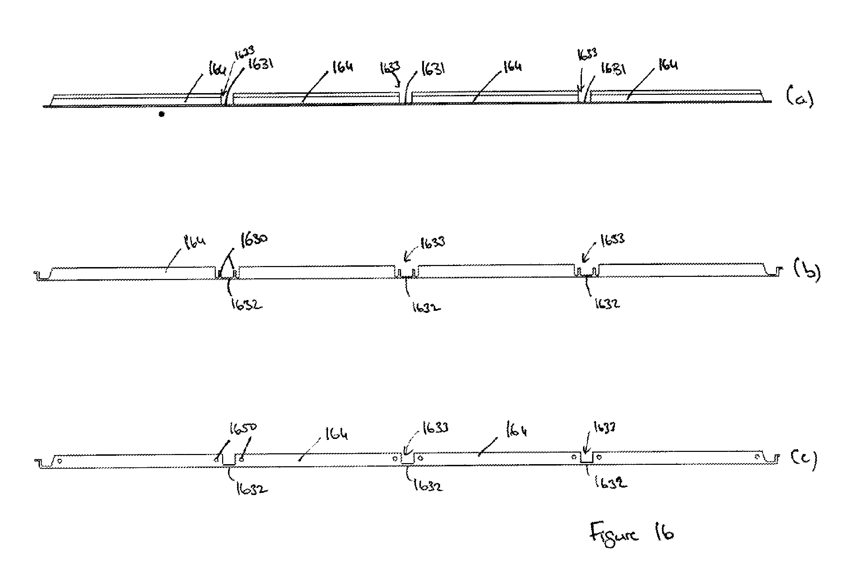

Figure 16(a)-(c) shows respective side views of a blades of the invention. In **Figure 16(a)** a series of blades 164 which can be used with the present invention. The blades 164 are carried on a common carrier in the form of an elongate implement support which may be a rod 1631. The rod 1631 may additionally function as structural support elements (such as those shown as item 813 in Figure 8). The embodiment of implements shown in Figure 16(a) are however adapted to be inserted through a mesh from underneath, with the blades 164 extending through the mesh. In such an arrangement the implements can be bonded to the mesh, but the implement support will prevent the blades detaching from the mesh as it will be trapped beneath the mesh. Bonding to the mesh is desirable in such an instance to ensure that the mesh and the implements move in tandem. As can be seen slots or grooves 1633 define the space between the adjacent implements.

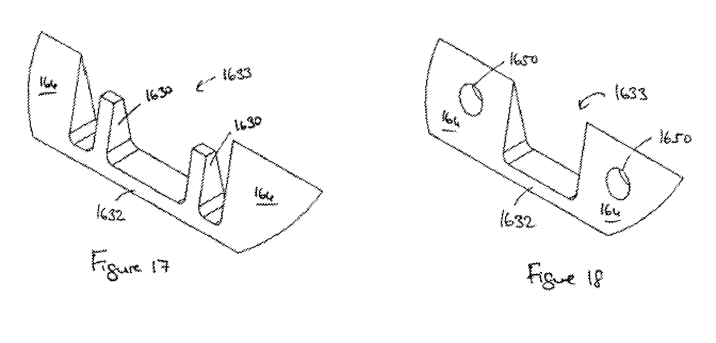

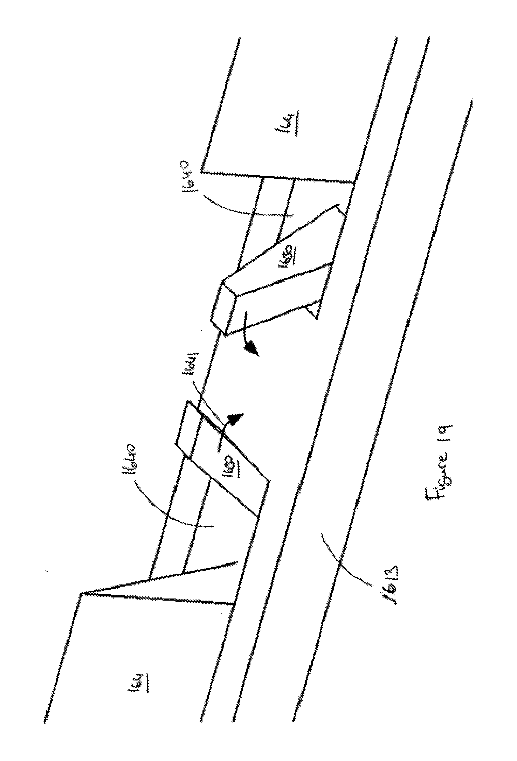

**Figure 16(b)** shows an array of implements in the form of blades 164 which are formed as a unitary piece, being joined by integrally formed connecting pieces 1632. Tabs 1630 are provided in the spaces between respective implements. An enlarged view of one pair of tabs 1630 is shown in **Figure 17****.** As shown in **Figure 19** the implements 164 are inserted through a structural member 1613 of a mesh portion from underneath. In particular the implements 164 extend through slots 1640 in the structural member and stand proud thereof. The mesh does not therefore interfere with the working action of the implements. As the arrows 1641 indicate, the tabs 1630 can be turned over, for example by bending the structural member thus securing the array of implements securely to the structural member. This means the implements move in tandem with the structural element. This prevents recession of the implements which would otherwise reduce the exposure of the implements above the structural element. It will be appreciated that a structural member is not required where the implements are secured directly to a mesh. If desired additional fixture (between the mesh and the implements and/or the tabs) can be achieved using a suitable adhesive material.

**Figure 16(c)** shows an array of implements in the form of blades 164 which are formed as a unitary piece, being joined by integrally formed connecting pieces 1632. Again spaces 1633 are provided between the blades 164. In this embodiment apertures 150 are formed in the blades 164. This is best seen from the enlarged view of part of the blade shown in **Figure 18****.**

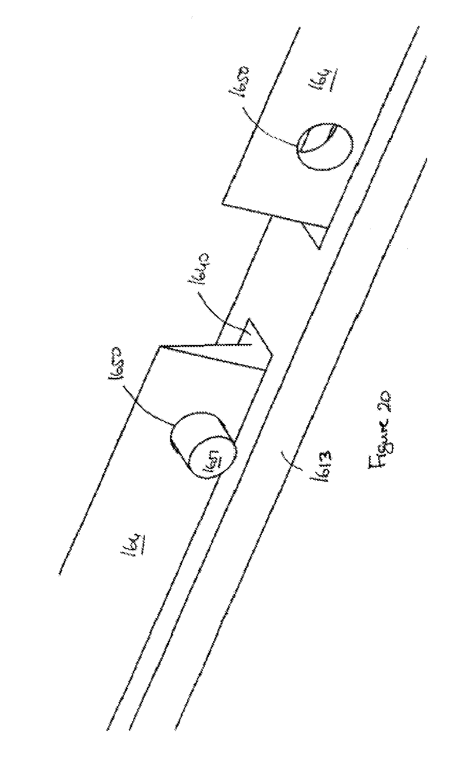

**Figure 20** shows how the blade of Figure 16(c) and Figure 18 engages with a mesh. The implements 164 are inserted through a structural member 1613 of a mesh portion from underneath. In particular the implements 164 extend through slots 1640 in the structural member and stand proud thereof. A pin 1651 can be inserted in each aperture 1650 thus securing the array of implements securely to the structural member. This means the implements move in tandem with the structural element. This prevents recession of the implements which would otherwise reduce the exposure of the implements above the structural element. It will be appreciated that a structural member is not required where the implements are secured directly to a mesh. If desired additional fixture (between the mesh and the implements and/or to fix the pin in place) can be achieved using a suitable adhesive material.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A collapsible treatment device for treating a target area of a vessel wall of a vessel within a human or animal body, comprising
(i) a mesh portion having a contracted configuration allowing travel within the vessel to the target area and an expanded configuration allowing treatment of the target area;
(ii) at least one treatment implement carried on the mesh portion; and further comprising
(iii) at least one protuberance which protects the vessel from contact with the implement during travel within the vessel.

2. A device according to Claim 1 wherein the protuberance is formed by the mesh portion.

3. A device according to Claim 1 or Claim 2 comprising a plurality of protuberances formed by the mesh portion

4. A device according to Claim 1 wherein the mesh portion comprises a main body which is annular.

5. A device according to any preceding claim wherein the mesh portion comprises at least one element which follows a tortuous path, the element resiliently deforming under expansion by decreasing tortuosity of its path.

6. A device according to any preceding claim further comprising a protective sheath for protecting the vessel from contact with the implement during travel within the vessel.

7. A device according to Claim 6 wherein said at least one protuberance which protects the vessel from contact with the implement during travel within the vessel is provided on the sheath.

8. A device according to any preceding claim wherein the protuberance deforms to expose the implement in the expanded configuration of the device.

9. A device according to anyone of Claim 1 to 5 wherein the protuberance houses the implement and the implement extends through the protuberance in the expanded configuration of the device.

10. A device according to any one of Claims 6 to 9 wherein the mesh portion is embedded within the sheath.

11. A device according to any one of Claims 6 to 9 wherein the mesh portion overlies the sheath.

12. A device according to any one of Claims 6 to 9 wherein the mesh portion underlies the sheath.

13. A device according to any one of Claims 6 to 9 wherein the mesh portion overlies the sheath and the protuberance extends from the sheath past the mesh portion.

14. A device according to any preceding claim wherein the device further comprises an expandable portion such as a balloon and the mesh portion is fitted over the expandable portion

15. A device according to any preceding claim wherein the mesh portion is machined from a tubular portion.

16. A device according to any preceding claim wherein the mesh portion is formed by moulding.

17. A device according to any preceding claims comprising at least two annular mesh portions.

18. A device according to any preceding claims comprising at least three annular mesh portions.

19. A device according to Claim 17 or 18 wherein the mesh portions are integrally formed.

20. A device according to any one of Claims 17 to 19 wherein each mesh portion forms a turn within a helix.

21. A device according to Claim 17 or Claim 18 wherein said annular mesh portions are not integrally formed but are joined by one or more separate elements.

22. A device according to Claim 21 wherein said one or more separate elements comprises a treatment implement or a carrier for a treatment implement.

23. A device according to any one of Claims 6 to 22 wherein the sheath forms an inflatable balloon.

24. A collapsible treatment device for treating a target area of a vessel wall of a vessel within a human or animal body, the device comprising:
an expandable mesh portion which is expandable from a contracted configuration allowing travel within the vessel to the target area to an expanded configuration allowing treatment of the target area, the mesh portion being constructed of a shape memory material which has memory for the contracted configuration and
at least one treatment implement carried on the mesh portion.

25. A device according to Claim 24 which has one or more of the additional features of Claims 1 to 23.

26. An expandable mesh for use with a collapsible treatment device for treating a target area of a vessel wall of a vessel within a human or animal body, the mesh comprising:
an expandable mesh portion which is expandable from a contracted configuration allowing travel within the vessel to the target area, to an expanded configuration allowing treatment of the target area, and
at least one treatment implement carried on the mesh portion, the mesh portion further comprising at least one protuberance which protects the vessel from contact with the implement during travel within the vessel.

27. An expandable mesh for use with a collapsible treatment device for treating a target area of a vessel wall of a vessel within a human or animal body, the mesh comprising:
(i) an expandable mesh portion which is expandable from a contracted configuration allowing travel within the vessel to the target area, to an expanded configuration allowing treatment of the target area;
(ii) at least one treatment implement carried on the mesh portion; and
(iii) a sheath provided on the mesh the sheath comprising at least one protuberance which protects the vessel from contact with the implement during travel within the vessel.
